# EUROPEAN PATENT SPECIFICATION

(11) **EP 1 347 298 B1**
(45) Date of publication and mention of the grant of the patent: **31.05.2006**
(21) Application number: 03006197.2
(22) Date of filing: 19.03.2003
(51) Int. Cl.: G01N 33/53

(54) **Immunoassay method, test strip for use therein, and immunoassay apparatus**
Immunologisches Verfahren, Teststreifen und Vorrichtung
Procédé de détermination immunologique, bande de test et appareil

(30) Priority: 19.03.2002 JP 2002077324
(43) Date of publication of application: 24.09.2003
(73) Proprietor: MATSUSHITA ELECTRIC INDUSTRIAL CO., LTD., Kadoma-shi, Osaka 571-8501 (JP)
(72) Inventor: Shigetoh, Nobuyuki, Kyotanabe-shi, Kyoto 610-0354 (JP)
(74) Representative: Grünecker, Kinkeldey, Stockmair & Schwanhäusser Anwaltssozietät

(56) References cited:
- EP-A- 0 158 973
- EP-A- 0 774 666
- EP-A- 1 033 575
- US-A- 5 206 144
- TOWBIN H ET AL: "NEOEPITOPE IMMUNOASSAY: AN ASSAY FOR HUMAN INTERLEUKIN 1BETA BASED ON AN ANTIBODY INDUCED CONFORMATIONAL CHANGE" JOURNAL OF IMMUNOASSAY, MARCEL DEKKER, BASEL, CH, vol. 17, no. 4, 1996, pages 353-369, XP001070211 ISSN: 0197-1522

## Description

### BACKGROUND OF THE INVENTION

The present invention relates to an immunoassay method for assaying an antigen, typically a protein antigen.

An antigen-antibody reaction is a reaction in which an antibody binds to a characteristic region (epitope) of an antigen. In a protein antigen, the characteristic region (epitope) is said to be made up of three to four amino acid residues. An antibody recognizing an intended three- or four-residue amino acid sequence and the conformation thereof in a protein antigen is specific to the protein antigen. Therefore, an antibody will not react falsely unless the sample is contaminated with an impurity having identical amino acid sequence and conformation to those of the antigen. Under normal circumstances, there is substantially no possibility for such a false reaction to occur. Thus, it is normally sufficient in many cases that an antibody recognizes the intended amino acid sequence and the conformation thereof in a protein antigen.

However, an antibody recognizing a site of an antigen may be useless in some cases where it is necessary to reliably recognize a slight structural difference. For example, when immunologically assaying hemoglobin A1c (hereinafter referred to as "HbA1c"), which is a glycosylated protein, an anti-HbA1c antibody needs to be capable of reliably recognizing the difference between HbA1c and hemoglobin A0 (hereinafter referred to as "HbA0"), which is a non-glycpsylated protein that always coexists with HbA1c. However, the difference is merely the presence/absence of fructose bound to the N-terminal of the amino acid β chain. Therefore, an anti-HbA1c antibody needs to be capable of binding to the N-terminal site of the amino acid β chain with fructose bound thereto. In other words, the epitope for the anti-HbA1c antibody needs to be the N-terminal site of the amino acid β chain of HbA1c with fructose bound thereto. A typical method for preparing such an antibody is to use an artificial immunogen obtained by artificially binding an intended epitope to a carrier protein. An antibody obtained in such a manner reliably recognizes the intended epitope. However, as is also the case with HbA1c, the intended epitope is actually often embedded in the protein antigen. Thus, by simply mixing together a protein antigen and an antibody, an antigen-antibody reaction may not occur in some cases.

In view of this problem, a protein antigen is conventionally thermally denatured so that an intended epitope embedded in the protein antigen is exposed. For example, Japanese Patent Publication for Opposition No. 7-23891 discloses a method in which a protein antigen is thermally denatured so that an intended epitope is exposed to use the thermally-denatured protein antigen as a sample in an assay.

However, while thermal denaturing is an effective method for exposing an intended epitope of a protein antigen, it is difficult to control the process and it necessitates a preliminary task of determining optimal conditions with respect to the temperature, the time, etc. Moreover, the optimal conditions may vary depending on the composition of the buffer solution in which the protein antigen is dissolved. Thus, conventional immunoassay methods for protein antigens are generally quite arduous.

Towbin et al., J. of Immunol. 1996, 17(4), 353-369 describes an antibody, mAb2, which only binds to a so-called neo-epitope in a complex of IL-1ß with another antibody, mAb1 (IL-1ß/mAb1-complex, IgG), but not to uncomplexed IL-1ß. However, the mAb2 binds to the precursor of IL-1ß, pro-IL-1ß, such that the authors concluded that the mAb2 recognizes a conformation of IL-1ß resembling that of pro-IL-1ß.

### SUMMARY OF THE INVENTION

The present invention has been made in view of the above, and has an object to provide an immunoassay method with various advantages such as a high assay precision and a high degree of convenience.

An immunoassay method of the present invention is an immunoassay method for assaying a protein antigen which is hemoglobin A1c, the method including the steps of: (a) providing a first antibody and a protein antigen which is hemoglobin A1c having, embedded therein, an epitope to which the first antibody can specifically bind; (b) contacting, to the protein antigen lgM serving as, a second antibody that binds to at least a portion of the protein antigen excluding the epitope so as to expose the epitope of the protein antigen; and (c) contacting the first antibody to the epitope of the protein antigen alter the step (b)

With the immunoassay method of the present invention, it is possible to precisely, or conveniently and rapidly, assay a protein with its epitope embedded therein. Moreover, it is possible to measure the total amount of protein to which the second antibody is bound, along with the amount of the protein antigen.

It is preferred that the second antibody is a monoclonal antibody.

In this way, the second antibody uniformly binds to a particular site of the protein antigen. Therefore, it is possible to stably expose the epitope in the protein antigen, thereby improving the precision in assaying the protein antigen, the detection sensitivity, and the reprodudbility of the assay result.

The first antibody may be immobilized on a, solid support.

The solid support may be a microtiter plate.

The solid support may be a test strip.

A test strip of the present invention is a test strip for use in an immunoassay method for assaying a protein antigen which is hemoglobin A1c, the test strip including: a base material; an antibody immobilizing section provided on the base material with a first antibody immobilized thereon, the first antibody being capable of specifically binding to an epitope located inside a protein antigen which is hemoglobin A1c; a sample dripping zone spaced apart from the antibody immobilizing section on the base material; and an impact imparting section located between the antibody immobilizing section and the sample dripping zone on the base member, and spaced apart from the antibody immobilizing section, the impact imparting section being impregnated with lgM serving as a second antibody that binds to at least a portion of the protein antigen excluding the epitope so as to expose the epitope of the protein antigen .

With the test strip of the present invention, as the sample solution containing the protein to be assayed (i.e., the protein antigen) is dripped onto the sample dripping zone, and the solvent of the sample solution is allowed to migrate, the labeled substance is bound to the protein antigen in the impact imparting section, and the epitope is exposed. Then, the protein antigen arrives at, and held in, the antibody immobilizing section. Thus, by optically measuring the antibody immobilizing section (e.g., by measuring the absorbance thereof), it is possible to obtain data such as the presence/absence, the amount, etc., of the protein antigen.

An immunoassay apparatus of the present invention is an immunoassay apparatus into which a test strip is introduced, the test strip including: a base material; a sample dripping zone; an antibody immobilizing section spaced apart from the sample dripping zone on the base material with a first antibody immobilized thereon, the first antibody being capable of specifically binding to an epitope located inside a protein antigen which is hemoglobin A1c; and an impact imparting section located between the antibody immobilizing section and the sample dripping zone on the base member, and spaced apart from the antibody immobilizing section, the impact imparting section being impregnated with a second antibody that binds to at least a portion of the protein antigen excluding the epitope so as to expose the epitope of the protein antigen, the immunoassay apparatus including: a sample dripping section for dripping a sample onto the sample dripping zone; and an optical measurement section for optically measuring the antibody immobilizing section.

With the immunoassay apparatus of the present invention, the process of assaying a protein with its epitope embedded therein can be automated using the test strip, thereby eliminating the need for the operator to learn any special skills for assaying a protein with its epitope embedded therein. Therefore, it is possible to quite conveniently assay a protein with its epitope embedded therein

### BRIEF DESCRIPTION OF THE DRAWINGS

FIG. **1** is a flow chart illustrating an immunoassay method of Embodiment 1 of the present invention.
FIG. **2** illustrates a test strip of the present invention.
FIG. **3** illustrates an assay apparatus of the present invention:
FIG. **4** illustrates the chemical structure of the epitope of HbA1c (fructose-VAL-HIS-LEU-THR-CYS).
FIG. **5** is a graph illustrating the effect of a guanidine treatment in an enzyme immunoassay on the binding between an anti-HbA1c antibody (F3A7) and free HbA1c.
FIG. **6** is a graph illustrating the effect of adding an auxiliary antibody (HbM4) in an enzyme immunoassay on the binding between an anti-HbA1c antibody (F3A7) and free HbA1c.
FIG. **7** schematically illustrates an assay system used in immunochromatography.

### DESCRIPTION OF THE PREFERRED EMBODIMENTS

The present invention relates to an immunoassay method for assaying an antigen, typically a protein antigen which is hemoglobin A1c. More particularly, the present invention relates to an immunoassay method using a modified protein antigen which is hemoglobin A1c obtained by modifying a protein antigen which is hemoglobin A1c that cannot react with an antibody because an intended epitope is embedded therein so that it can effectively react with the antibody.

### EMBODIMENT 1

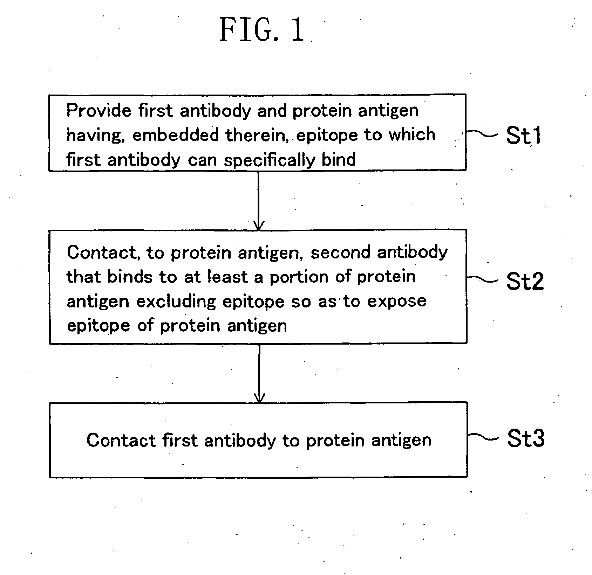

Embodiments of the present invention will now be described with reference to the drawings. FIG. **1** is a flow chart illustrating an immunoassay method of the present embodiment.

The immunoassay method of the present invention is an immunoassay method for assaying said protein antigen, and includes three steps as illustrated in FIG. 1. Specifically, the immunoassay method includes: step St1 of providing a first antibody and the protein antigen having, embedded therein, the epitope to which the first antibody can specifically bind; step St2 of contacting, to the protein antigen, said second antibody that binds to at least a portion of the protein antigen excluding the epitope so as to expose the epitope of the protein antigen; and step St3 of contacting the first antibody to the protein antigen after step St2.

These steps will now be described in greater detail.

First, in step St1, a first antibody and the protein that is recognized as an antigen by the first antibody (i.e., a protein antigen) are provided. The combination of the protein antigen and the first antibody is not limited to any particular combination, and may be any combination of a protein which is hemoglobin A1c that the operator wishes to assay (i.e., a target protein which is hemoglobin A1c) and an antibody that recognizes the protein. While the protein antigen may be any protein which is hemoglobin A1c, specific examples of other proteins for illustration the invention include those that may be assayed in medical and diagnostic fields, such as protamines, mucoproteins, glycoproteins, globulins, albumins, phosphoproteins, histones, lipoproteins, chromoproteins, and nucleoproteins.

Then, in step St2, the second antibody (auxiliary antibody) that binds to at least a portion of the protein antigen excluding the epitope is contacted to the protein antigen so as to expose the epitope of the protein antigen. The second antibody is an antibody that gives an impact on the protein antigen. The term "impact" as used herein refers to an influence that causes a change in the conformation of the protein antigen. By contacting the second antibody to the protein antigen, the intended epitope embedded in the protein antigen is exposed so that the intended epitope can be accessed by an antibody that specifically binds to the intended epitope, thereby allowing for immunological reaction between the protein antigen and the antibody.

The second antibody (auxiliary antibody) is typically a monoclonal antibody, and can easily be prepared by those skilled in the art by using a method well known in the art, such as a hybridoma technique described in Koehler and Milstein (Nature 256:495 [1975]), a human B cell hybridoma technique described in Kosbor, et al., 1983, Immunol. Today 4:72 and Cote, et al., 1983 (Proc. Natl. Acad. Sci. USA, 80:2026), and an EBV hybridoma technique described in Cole, et al., MONOCLONAL ANTIBODIES AND CANCER THERAPY, Alan Riss Inc., New York, NY, pp.77-96 [1985].

A method for preparing a monoclonal antibody will now be described briefly. First, an appropriate animal is immunized with a selected protein antigen. After immunization, the spleen is taken out from the animal, and spleen cells are fused with immortalized myeloma cells under selected conditions. Then, the obtained cells are separated into clones, and the supernatant of each clone is examined for the production of an antibody that binds to the protein antigen. Then, it is examined as to whether it promotes the binding between the first antibody and the epitope to which the first antibody binds, so as to prepare the auxiliary antibody. In the immunization process, the protein antigen may be bound to a carrier substance such as bovine serum albumin (BSA) or keyhole limpet hemocyanin (KHL), for example, so that the antibody is produced more reliably. The use of such a carrier substance is known to those skilled in the art.

The technique for producing an antibody is well known in the art, as described in, for example, Goding, et al., MONOCLONAL ANTIBODIES: PRINCIPLES AND PRACTICE (2nd Edition) Acad. Press, N. Y.

The second antibody (auxiliary antibody) is used by being mixed with the protein antigen in an amount sufficient to promote the reaction between the first antibody and the epitope to which the first antibody binds. Specifically, in order to promote the reaction between the first antibody and the epitope to which the first antibody binds, it is preferred that the second antibody and the protein antigen are mixed together so that the ratio between the molecular count of the second antibody and that of the protein antigen is 1:10 to 10:1.

Moreover, as necessary, the second antibody may be labeled with any labeling method known in the art, e.g., enzyme labeling, dye labeling, magnetic labeling, radioactive labeling, or labeling with colored particles (a gold colloid, a latex, etc.).

While the second antibody that binds to at least a portion of the protein antigen excluding the epitope is used for giving an impact on the protein antigen in the present embodiment, this can alternatively be done by, for example, a chemical process of adding a chemical agent such as dithiothreitol or guanidine, or a protein such as an enzyme, to a solution containing the protein antigen, or a physical process of performing a photoirradiation, an ultrasonic treatment, or the like, on a solution containing the protein antigen.

When employing the chemical process, a chemical agent called "chaotropic agent" may be used at an appropriate concentration. Typical chaotropic agents that can suitably be used with the present invention include guanidine, urea, sodium dodecyl sulfate, deoxycholate, a bile salt, an organic solvent (methanol, propanol, acetonitrile, etc.), potassium thiocyanate, a nonionic surfactant, mercaptoethanol, and dithiothreitol. These chemical agents are used by being contacted to the protein antigen under a room temperature condition, i.e., a non-heated condition, and are used at a sufficient concentration for exposing the intended epitope in the protein antigen. With guanidine, for example, the intended epitope in the protein antigen can be exposed normally by contacting about 1 M or more, preferably about 3 M or more, of guanidine to the protein antigen for about five minutes to several hours.

Note that the chemical and physical processes as described above cause a substantial change in the conformation of the protein antigen. Moreover, in some cases, these processes are performed at temperatures significantly higher than the optimal temperature for the protein antigen. Therefore, the activity and the conformation of the protein antigen may change significantly. In such a case, the antigen-antibody reaction occurring in step St3 to be described below is likely to be quite different from that occurring under an in-vivo environment.

On the other hand, with the method of the present embodiment in which the second antibody (auxiliary antibody) that binds to at least a portion of the protein antigen excluding the epitope is used, it is possible to cause a relatively slight change in the conformation of the protein antigen without denaturing the protein antigen. Moreover, this method is performed in the vicinity of the optimal temperature for the protein antigen. Therefore, it is unlikely that the conformation of the protein antigen changes significantly. Thus, the antigen-antibody reaction occurring in step St3 to be described later will be quite similar to that occurring under an in-vivo environment, whereby it is possible to obtain assay results that better reflect the in-vivo environment.

Next, in step St3, the first antibody is contacted to the protein antigen. In this process, the first antibody binds to the intended epitope of the protein antigen, which has been exposed in step St2 described above.

More specifically, in the immunoassay method of the present embodiment, the first antibody is immobilized on a solid support. For example, the solid support may suitably be a test strip of a nitrocellulose membrane, a cellulose acetate membrane, glass fiber filter paper, non-woven fabric, or the like. Alternatively, the solid support may suitably be a microtiter plate. The microtiter plate may be made of polystyrene, polyvinyl, polycarbonate, polypropylene, a silicon material, a glass material, a dextran material, or the like.

In the immunoassay method of the present embodiment, steps St1 to St3 as described above are performed, after which the protein antigen is assayed by a method known to those skilled in the art, e.g., enzyme immunoassay (ELISA), fluoroimmunoassay (FIA), immunoturbidimetry, immunonephelometry, or immunochromatography. Among others, enzyme immunoassay, which is sensitive, and immunochromatography, which is convenient and rapid, are more preferably used to further improve the assay precision and the convenience of the immunoassay method of the present embodiment.

With the immunoassay method of the present embodiment, it is possible to precisely, or conveniently and rapidly, assays the protein HbA1c with its characteristic epitope embedded therein .

Moreover, when the second antibody is used as a material for giving an impact on the protein antigen, it is possible to measure the total amount of protein to which the second antibody is bound, along with the amount of the protein antigen.

### EMBODIMENT 2

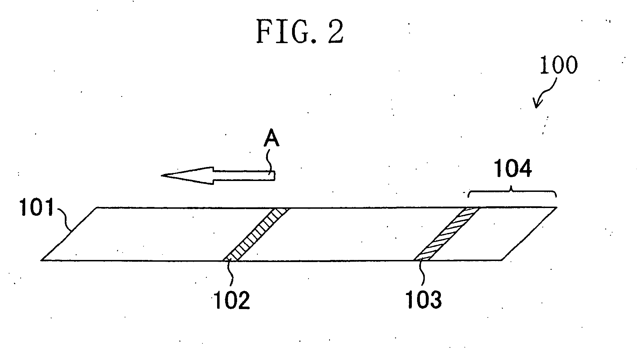

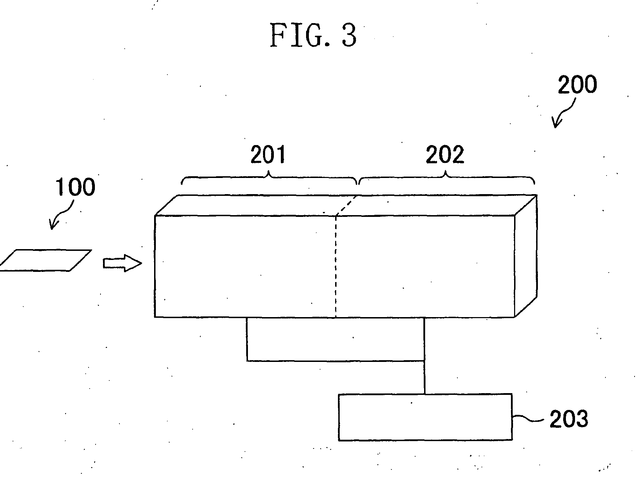

Next, a test strip and an assay apparatus for use in the immunoassay method of Embodiment 1 will be described with reference to the drawings. FIG. **2** illustrates a test strip for use in the immunoassay method of Embodiment 1, and FIG. **3** illustrates an assay apparatus for use in the immunoassay method of Embodiment 1.

### TEST STRIP

As illustrated in FIG. **2**, a test strip **100** is made of a base material **101**, and has an antibody immobilizing section **102,** an impact imparting section **103**, and a sample dripping zone **104**.

The base material **101** is made of a material capable of absorbing the solvent of the sample solution containing the protein to be assayed (i.e., the protein antigen), and may be a nitrocellulose membrane, a cellulose acetate membrane, glass fiber filter paper, non-woven fabric, or the like.

An antibody that recognizes the protein antigen (i.e., the first antibody of Embodiment 1) is immobilized on the antibody immobilizing section **102.**

The impact imparting section **103** is impregnated with a labeled substance that gives an impact on the protein antigen (e.g., the second antibody of Embodiment 1 labeled with a gold colloid).

The sample dripping zone **104** is a portion of the test strip **100** onto which the sample solution containing the protein to be assayed (i.e., the protein antigen) is dripped.

As the sample solution containing the protein to be assayed (i.e., the protein antigen) is dripped onto the sample dripping zone **104**, and the solvent of the sample solution is allowed to migrate in the direction of arrow A in FIG. **2**, the labeled substance is bound to the protein antigen in the impact imparting section **103**, and the epitope is exposed. Then, the protein antigen arrives at, and held in, the antibody immobilizing section **102.** Thus, by optically measuring the antibody immobilizing section **102** (e.g., by measuring the absorbance thereof), it is possible to obtain data such as the presence/absence, the amount, etc., of the protein antigen.

In the test strip **100** of the present embodiment, a labeled substance that gives an impact on the protein antigen is immobilized on the impact imparting section **103**. Specifically, the labeled substance that gives an impact on the protein antigen may be the second antibody of Embodiment 1 labeled with a gold colloid, but is not limited thereto. Alternatively, one of the chaotropic agents listed in Embodiment 1 and an antibody that binds to the protein antigen but does not give an impact may be immobilized on the impact imparting section **103.**

### ASSAY APPARATUS

As illustrated in FIG. **3**, an assay apparatus **200** includes a sample dripping section **201** for dripping a sample solution onto the sample dripping zone **104** of the test strip **100** being inserted into the assay apparatus **200,** an optical measurement section **202** for optically measuring the antibody immobilizing section **102** of the test strip **100,** and a control/analysis section **203** electrically connected to the sample dripping section **201** and the optical measurement section **202**.

As the test strip **100** is inserted into the assay apparatus **200**, the test strip **100** is carried into the sample dripping section **201**. The sample dripping section **201** includes a tank storing the sample solution and another tank storing the solvent of the sample solution. When the control/analysis section **203** detects that the test strip **100** has been carried into the sample dripping section **201,** the control/analysis section **203** instructs the sample dripping section **201** to drip the sample solution onto the sample dripping zone **104.** After the sample solution is dripped onto the sample dripping zone **104,** the solvent of the sample solution is dripped from the sample dripping section **201** onto the sample dripping zone **104**, and the sample contained in the sample solution is allowed to migrate through the test strip **100**.

Then, after the passage of a predetermined amount of time, which has been set in the control/analysis section **203,** the test strip **100** is carried into the optical measurement section **202**. Then, the antibody immobilizing section **102** of the test strip **100** is optically measured. Data obtained through the measurement is passed to the control/analysis section **203** for calculation, analysis, etc.

With the assay apparatus **200** of the present invention, the steps of the immunoassay method of Embodiment 1 can be automated using the test strip **100**, thereby eliminating the need for the operator to learn any special skills for carrying out the immunoassay method of Embodiment 1. Therefore, it is possible to carry out the immunoassay method of Embodiment 1 quite conveniently.

### EXAMPLES

The present invention will now be described in greater detail by way of an example. The following example is provided solely for the purpose of illustration and should not be taken to limit the scope of the present invention.

In the following example, HbA1c and an anti-HbA1c antibody are used as the protein antigen and the first antibody, respectively. Moreover, guanidine and the second antibody (auxiliary antibody) are used as means for exposing the epitope, and the assay is based on enzyme immunoassay and immunochromatography. Note that the second antibody is referred to as "auxiliary antibody" in the following example.

### PREPARATION OF ANTI-HbA1c ANTIBODY

A monoclonal antibody was prepared as follows, which is an anti-HbA1c antibody that recognizes, as the epitope, the characteristic structure of HbA1c that distinguishes itself from HbA0.

### 1. Immunization Of Mouse

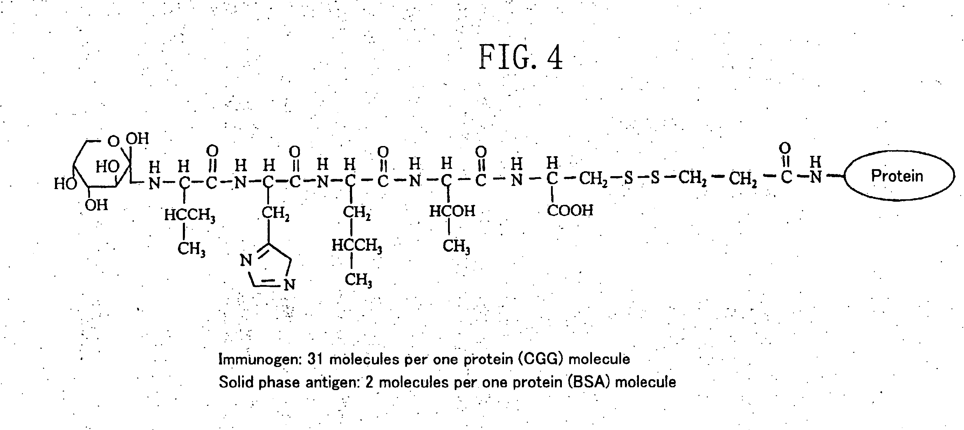

A CGG conjugate was prepared, in which 31 molecules of the HbA1c epitope having a chemical structure as illustrated in FIG. **4** (fructose-VAL-HIS-LEU-THR-CYS) are bound per one molecule of chicken γ-globulin (CGG), and the CGG conjugate was used as an artificial immunogen for subject mice. One hundred µL of the prepared artificial immunogen (CGG conjugate-adjuvant mixture) was injected into the peritoneal cavity of five mice (Balb/c) about eight weeks old. After 77 days from the immune injection, 50 to 100 µL of blood was collected from the mice through the ophthalmic vein into a centrifuge tube. Sera obtained through centrifugation were subjected to an evaluation for the antibody titer by ELISA, confirming that the anti-HbA1c antibody was produced in all mice. In the ELISA evaluation, a BSA conjugate, in which two molecules of the HbA1c epitope having a chemical structure as illustrated in FIG. **4** (fructose-VAL-HIS-LEU-THR-CYS) were bound per one molecule of bovine serum albumin (BSA), and native HbA1c were used as solid phase antigens.

Those mice that had been evaluated to have particularly high titers were boosted (injected with a weak immunogen) for swelling the spleen. As the immunogen, a 1 mg/mL CGG conjugate solution obtained by dilution with a phosphate buffered solution (PBS) was used as it is, without adding an adjuvant thereto.

### 2. Cell Fusion

Spleen cells were taken out from the mice after three days from boosting, and fused with a mouse-myeloma-derived cell line (P3X63-Ag8.653) by an ordinary method using polyethylene glycol having an average molecular weight of 1,500. Using spleen cells from the same mice as feeder cells (cells that feed growth factor), the fused cells were cultured on an HAT culture medium containing 15% fetal calf serum (hereinafter "FCS") on two 96-well plates. After one week, the culture medium was replaced with a fresh HAT culture medium containing 15% FCS.

### 3. Cloning

The antibody titer was evaluated by ELISA to select five wells of highest titers. The culture was diluted to a concentration such that each well contains one cell (limiting dilution), and was fractionated into individual wells of five 96-well microplates. Thymocytes of five-week-old mice (Balb/c) were used as feeder cells to promote the initial growth. The culturing process was continued while increasing the plate size. The antibody titer evaluation of the supernatant by ELISA was repeated as necessary to finally screen the cell colonies for those exhibiting high titers for HbA1c and exhibiting desirable growth, and the culturing process was continued to obtain a concentration of 5x105 cells/mL with a volume of 200 ml. The finally selected cell cultures were centrifuged to separate the supernatant, frozen at -80°C while being floated in 1 mL of a solution of FCS:dimethyl sulfoxide = 9:1 at a concentration of 5x10⁵ cells/mL, and placed in liquid nitrogen for long-term storage.

Before use, a monoclonal antibody was purified from the cell culture supernatant by affinity chromatography using a Protein-A Sepharose gel (from Pharmacia Corporation). A test with Mouse Monoclonal Typing Kit (from The Binding Site Limited) confirmed that the monoclonal antibody was of the IgG type, and the monoclonal antibody was designated "F3A7". While the antibody (F3A7) bound to a BSA conjugate including the epitope bound to BSA and to HbA1c immobilized on a plate, but did not bind to free HbA1c in the solution. Moreover, F3A7 did not substantially bind to HbA0 irrespective of the state of HbA0 (i.e., whether it is immobilized or free).

### PREPARATION OF AUXILIARY ANTIBODY

An auxiliary antibody capable of exposing the HbA1c epitope was prepared as follows. A human hemoglobin prepared to a concentration of 1 mg/ml was used as an immunogen to immunize mice, and the antibody titer was evaluated as described above. After 80 days from the initial immunization, the mice were boosted as described above, and the cell fusion process was performed. The well screening after the fusion was performed based on a measurement under the following ELISA conditions.

The anti-HbA1c antibody (F3A7) prepared to a concentration of 0.1 mg/ml was injected into a 96-well ELISA plate in a volume of 100 µl/well, and left standing overnight at 4°C to be immobilized. The plate was blocked with 200 µl/well of 1% BSA-PBS (at room temperature for 30 minutes), and then washed with PBS three times. Then, 50 µl/well of a culture supernatant was introduced into the plate, after which 50 µl/well of a PBS solution of HbA1c was added so as to obtain a final concentration of 10⁻⁷ M (total volume: 100 µl/well). The plate was left standing at room temperature for three hours, and then washed with PBS three times. A peroxidase-labeled anti-hemoglobin antibody (from Bethyl Laboratories, Inc.) prepared to a concentration of 0.2 µg/ml was added to the plate in a volume of 100 µl/well, and the plate was left standing at room temperature for 30 minutes and washed with PBS three times. Then, a phosphate/citrate buffered solution of o-phenylenediamine (pH=5) prepared to a concentration of 4 mg/ml was added to the plate in a volume of 100 µl/well, and an enzyme reaction was allowed to take place for five minutes. After the reaction was stopped with 4 N sulfuric acid, the absorbance at 492 nm was measured by using a plate reader.

For those wells for which color development was observed through the ELISA process described above, a cloning process was performed in a manner similar to that for the anti-HbA1c antibody to obtain a purified monoclonal antibody. A test with Mouse Monoclonal Typing Kit (from The Binding Site Limited) confirmed that the monoclonal antibody was of the IgM type, and the monoclonal antibody was designated "HbM4". HbM4 bound both to HbA0 and to HbA1c. The cell line producing HbM4 was deposited with International Patent Organism Depositary, National Institute of Advanced Industrial Science and Technology on January 30, 2003 (Accession Number: FERM BP-8286).

### ASSAY FOR FREE HbA1c BY ENZYME IMMUNOASSAY

### 1. Assay With Guanidine

Guanidine hydrochloride was added to a PBS solution of HbA1c having a concentration of 10⁻⁴ M so that the final concentration was 3 M, and the mixture was left standing at room temperature for three hours. The solution was diluted with PBS to prepare a series of HbA1c dilutions of 10⁻⁵ M, 10⁻⁶ M, 10⁻⁷ M, 10⁻⁸ M, 10⁻⁹ M, 10⁻¹⁰ M and 10⁻¹¹ M. A similar series of dilutions, but without guanidine, was also prepared for the purpose of comparison.

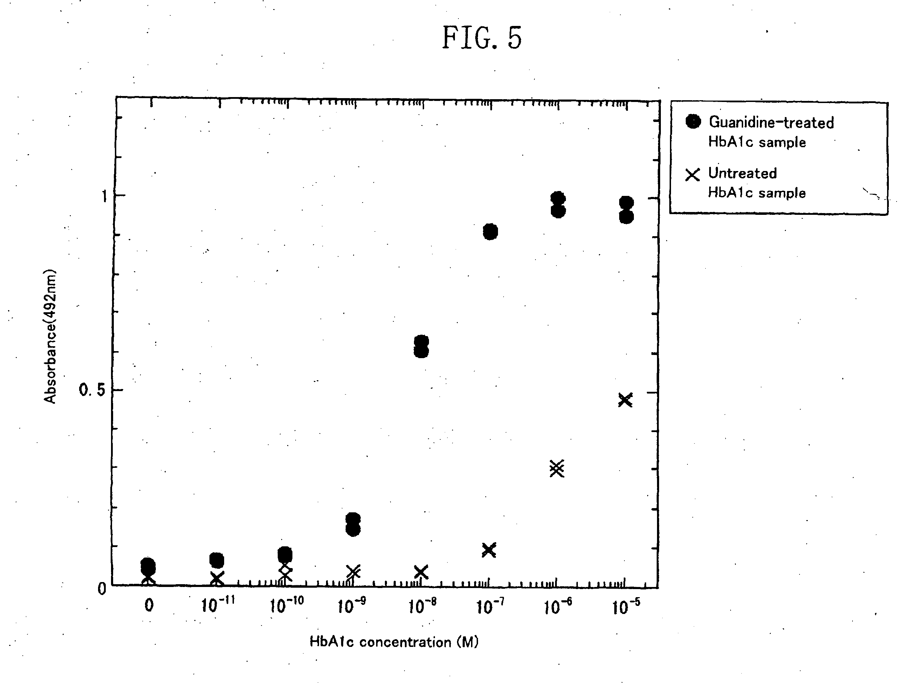

The anti-HbA1c antibody (F3A7) prepared to a concentration of 0.1 mg/ml was injected into a 96-well ELISA plate in a volume of 100 µl/well, and left standing overnight at 4°C to be immobilized. The plate was blocked with 200 µl/well of 1% BSA-PBS (at room temperature for 30 minutes), and then washed with PBS three times. Then, the series of dilutions of HbA1c prepared as described above was added to the plate at 100 µl/well, and left standing at room temperature for three hours and washed with PBS three times. A peroxidase-labeled anti-hemoglobin antibody (from Bethyl Laboratories, Inc.) prepared to a concentration of 0.2 µg/ml was added to the plate in a volume of 100 µl/well, and the plate was left standing at room temperature for 30 minutes and washed with PBS three times. Then, a phosphate/citrate buffered solution of o-phenylenediamine (pH=5) prepared to a concentration of 4 mg/ml was added to the plate in a volume of 100 µl/well, and an enzyme reaction was allowed to take place for five minutes. After the reaction was stopped with 4 N sulfuric acid, the absorbance at 492 nm was measured by using a plate reader. The results are shown in FIG. 5. As shown in FIG. 5, non-guanidine-treated HbA1c reacted with the anti-HbA1c antibody (F3A7) only at concentrations of about 10⁻⁷ M or more, whereas guanidine-treated HbA1c reacted with F3A7 starting from a concentration of about 10⁻⁹ M. This indicates that the chemical impact by the addition of guanidine caused the epitope of HbA1c to be exposed to a degree effective for reacting with an antibody.

### 2. Assay With Auxiliary Antibody (HbM4)

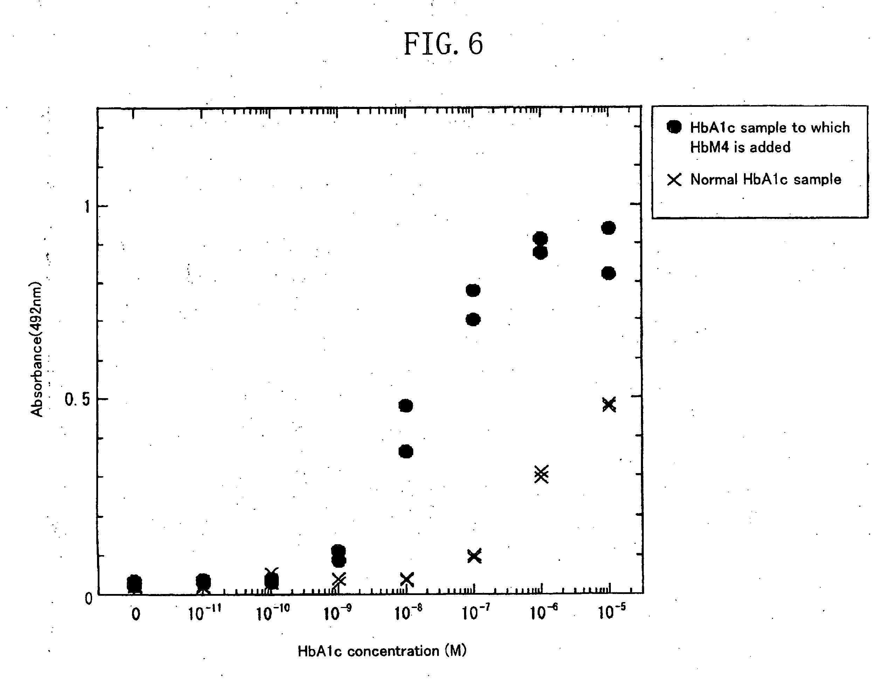

A 0.02 mg/ml PBS solution of HbM4 (final concentration: 0.01 mg/ml) was prepared, and used as an auxiliary antibody. A series of HbA1c dilutions at concentrations of 2x10⁻⁵ M to 2x10⁻¹¹ M was used so that the final concentrations were 10⁻⁵ M to 10⁻¹¹M, as in the assay described above. Fifty µl of the series of HbA1c dilutions and 50 µl of the HbM4 solution were placed into each well of a plate that had been immobilized and blocked as in the assay described above, and left standing at room temperature for three hours and washed with PBS three times. Then, as in the assay described above, the reaction with a peroxidase-labeled anti-hemoglobin antibody, and the enzyme reaction with the addition of a matrix were performed, and the absorbance at 492 nm was measured. The results are shown in FIG. 6. As shown in FIG. 6, without the auxiliary antibody, HbA1c reacted with the anti-HbA1c antibody (F3A7) only at concentrations of about 10⁻⁷ M or more, whereas with the auxiliary antibody, HbA1c reacted with F3A7 starting from a concentration of about 10⁻⁹ M. This indicates that the addition of HbM4 caused the epitope of HbA1c to be exposed to a degree effective for reacting with an antibody.

### ASSAY OF FREE HbA1c BY IMMUNOCHROMATOGRAPHY

### 1. Assay With Guanidine

An anti-hemoglobin antibody that did not exhibit an epitope-exposing effect (Hb4-1, prepared separately) was adsorbed onto the surface of a gold colloid having a grain diameter of 20 nm under a condition of pH 9 to prepare a gold-colloid-labeled hemoglobin antibody (Au-Hb4-1). A solution containing the labeled antibody was adjusted, with a tris buffered solution of pH 8.2 containing 1% BSA, so that the absorbance at 520 nm was 5.0. The adjusted solution was fractionated into 25-µl portions, and freeze-dried for storage at 4°C until use.

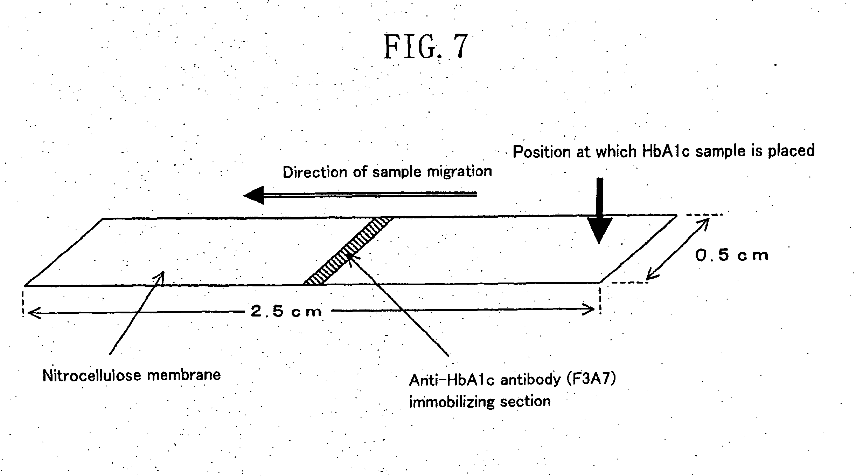

On the other hand, the anti-HbA1c antibody (F3A7) was prepared in a PBS solution to a concentration of 1 mg/ml. As illustrated in FIG. 7, F3A7 was applied in a straight line on a nitrocellulose membrane (from Millipore Corporation) having a width of 0.5 cm and a length of 2.5 cm, and air-dried to be immobilized on the membrane.

In the assay, a guanidine-treated HbA1c solution having a concentration of 10⁻⁷ M, which is similar to that used in the enzyme immunoassay described above, was used, along with a non-guanidine-treated HbA1 c solution having the same concentration for the purpose of concentration.

First, 25 µl of the guanidine-treated sample was mixed with the gold-colloid-labeled hemoglobin antibody (Au-Hb4-1), which had been prepared, freeze-dried and stored. After confirming complete thawing of freeze-dried Au-Hb4-1, the solution was placed at one end of the membrane illustrated in FIG. 7. The solution was allowed to migrate by capillary action through the membrane to reach the portion where F3A7 was immobilized, when the color of the gold colloid developed. After five minutes from the placement of the sample, the absorbent of the immobilizing section at 520 nm was measured by a scanning densitometer (CS-9300 from Shimadzu Corporation) to be 0.35. Then, the non-guanidine-treated HbA1c sample was similarly allowed to migrate through the membrane for the purpose of comparison, but the color development at the immobilizing section was not observed. With this sample, the absorbance of the immobilizing section at 520 nm was 0.02, substantially equal to the background absorbance of portions of the membrane other than the immobilizing section.

### 2. Assay With Auxiliary Antibody (HbM4)

The auxiliary antibody HbM4 was adsorbed onto the surface of a gold colloid as in the assay with guanidine described above to prepare gold-colloid-labeled HbM4 (Au-HbM4). An immobilizing membrane on which F3A7 was immobilized was used, as in the assay described above.

Twenty five µl of a 10⁻⁷ M HbA1c solution (a normal HbA1c solution without special treatment) was mixed with gold-colloid-labeled HbM4 (Au-HbM4), which had been freeze-dried and stored. After confirming complete thawing of freeze-dried Au-HbM4, the solution was placed at one end of the immobilizing membrane, and the solution was allowed to migrate through the membrane. Even though the HbA1c sample had not been subjected to any special treatment, color development at the immobilizing section was observed. After five minutes from the placement of the sample, the absorbance of the immobilizing section at 520 nm was 0.55. In the case of Au-Hb4-1 described above, color development was not observed with an untreated HbA1c sample. Therefore, it can be seen that Au-HbM4 is a useful material that has the function as a labeled antibody contributing to the color development and also has the epitope-exposing function.

As described above, with the immunoassay method of the present invention, even a protein having an epitope embedded therein, which is difficult to assay, can easily be assayed without being thermally denatured.

Moreover, the immunoassay method of the present invention can be applied to various basic assay methods existing in the prior art, such as enzyme immunoassay, fluoroimmunoassay, agglutination immunoassay, immunonephelometry, and immunochromatography. Among others, the application of the present invention to immunochromatography is quite effective as it allows for maximum utilization of the advantageous features of immunochromatography, i.e., being convenient and rapid.

Thus, the present invention provides an immunoassay method with various advantages such as a high assay precision and a high degree of convenience.

## Claims

1. An immunoassay method for assaying a protein antigen, which is hemoglobin A1 c, the method comprising the steps of:
(a) providing a first antibody and a protein antigen, which is hemoglobin A1 c, having, embedded therein, an epitope to which the first antibody can specifically bind;
(b) contacting, to the protein antigen, lgM serving as a second antibody that binds to at least a portion of the protein antigen excluding the epitope so as to expose the epitope of the protein antigen; and
(c) contacting the first antibody to the epitope of the protein antigen after the step (b).

2. An immunoassay method of claim 1, wherein the second antibody is a monoclonal antibody.

3. An immunoassay method of claim 1, wherein the first antibody is immobilized on a solid support.

4. An immunoassay method of claim 3, wherein the solid support is a microtiter plate.

5. An immunoassay method of claim 3, wherein the solid support is a test strip.

6. A test strip for use in an immunoassay method for assaying a protein antigen, which is hemoglobin A1 c, the test strip comprising:
a base material;
an antibody immobilizing section provided on the base material with a first antibody immobilized thereon, the first antibody being capable of specifically binding to an epitope located inside a protein antigen, which is hemoglobin A1c; a sample dripping zone spaced apart from the antibody immobilizing section on the base material; and
an impact imparting section located between the antibody immobilizing section and the sample dripping zone on the base member, and spaced apart from the antibody immobilizing section, the impact imparting section being impregnated with IgM serving as a second antibody that binds to at least a portion of the protein antigen excluding the epitope, so as to expose the epitope of the protein antigen.

7. An immunoassay apparatus into which a test strip is introduced, the test strip including:
a base material;
a sample dripping zone;
an antibody immobilizing section spaced apart from the sample dripping zone on the base material with a first antibody immobilized thereon, the first antibody being capable of specifically binding to an epitope located inside a protein antigen, which is hemoglobin A1c; and
an impact imparting section located between the antibody immobilizing section and the sample dripping zone on the base member, and spaced apart from the antibody immobilizing section, the impact imparting section being impregnated with IgM serving as a second antibody that binds to at least a portion of the protein antigen excluding the epitope, so as to expose the epitope of the protein antigen the immunoassay apparatus comprising:
a sample dripping section for dripping a sample onto the sample dripping zone; and
an optical measurement section for optically measuring the antibody immobilizing section.

## Patentansprüche

1. Ein Immunoassay-Verfahren zum Nachweis eines Protein-Antigens, welches Hämoglobin A1c ist, wobei das Verfahren die folgenden Schritte umfasst:
(a) Zur-Verfügung-Stellen eines ersten Antikörpers und eines Protein-Antigens, welches Hämoglobin A1c ist, mit - eingebettet darin - einem Epitop, an welches der erste Antikörper spezifisch binden kann;
(b) In-Kontakt-Bringen eines lgM, dienend als zweiter Antikörper, mit dem Protein-Antigen, wobei das lgM an zumindest einen Teil des Protein-Antigens bindet ausschließend das Epitop, um das Epitop des Protein-Antigens zu exponieren; und
(c) In-Kontakt-Bringen des ersten Antikörpers mit dem Epitop des Protein-Antigens nach dem Schritt (b)

2. Ein Immunoassay-Verfahren von Anspruch 1, wobei der zweite Antikörper ein monoklonaler Antikörper ist.

3. Ein lmmunoassay-Verfahren gemäß Anspruch 1, wobei der erste Antikörper auf einer festen Unterlage immobilisiert ist.

4. Ein Immunoassay-Verfahren nach Anspruch 3, wobei die feste Unterlage eine Mikrotiterplatte ist.

5. Ein Immunoassay-Verfahren gemäß Anspruch 3, wobei die feste Unterlage ein Teststreifen ist.

6. Ein Teststreifen zur Anwendung in einem Immunoassay-Verfahren zum Nachweis eines Protein-Antigens, welches Hämoglobin A1c ist, wobei der Teststreifen folgendes umfasst:
ein Basismaterial;
einen Antikörper-immobilisierenden Abschnitt, bereitgestellt auf dem Basismaterial, mit einem ersten Antikörper darauf immobilisiert, wobei der erste Antikörper in der Lage ist, spezifisch an ein Epitop, lokalisiert innerhalb eines Protein-Antigens, zu binden, welches Hämoglobin A1c ist;
eine Proben-Ablagezone, räumlich getrennt von dem Antikörper immobilisierenden Abschnitt auf dem Basismaterial; und
einen Auswirkung-verleihenden Abschnitt, lokalisiert zwischen dem Antikörper immobilisierenden Abschnitt und der Proben-Ablagezone auf dem Basismaterial und räumlich getrennt von dem Antikörper-immobilisierenden Abschnitt, wobei der Auswirkung-verleihende Abschnitt mit lgM imprägniert wird, welcher als zweiter Antikörper dient, der zumindest an einen Teil des Protein-Antigens bindet ausschließend das Epitop, um das Epitop des Protein-Antigens zu exponieren.

7. Ein Immunoassay-Apparat, in welchem ein Teststreifen eingebracht wird, wobei der Teststreifen folgendes enthält:
ein Basismaterial;
eine Proben-Ablagezone;
einen Antikörper-immobilisierender Abschnitt, räumlich getrennt von der Proben-Ablagezone auf dem Basismaterial, mit einem ersten Antikörper darauf immobilisiert, wobei der erste Antikörper in der Lage ist, spezifisch an ein Epitop, lokalisiert innerhalb eines Protein-Antigens, zu binden, welches Hämoglobin A1c ist; und
einen Auswirkung-verleihenden Abschnitt, lokalisiert zwischen dem Antikörper-immobilisierenden Abschnitt und der Proben-Ablagezone auf dem Basismaterial und räumlich getrennt von dem Antikörper-immobilisierenden Abschnitt, wobei der Auswirkung verleihende Abschnitt imprägniert ist mit IgM, welcher als zweiter Antikörper dient, der an zumindest einen Teil des Protein-Antigens bindet ausschließend das Epitop, um das Epitop des Protein-Antigens zu exponieren, wobei der Immunoassay-Apparat folgendes umfasst:
einen Proben-Ablage-Abschnitt zum Ablegen einer Probe auf der Proben-Ablagezone; und
einen optischen Messabschnitt zum optischen Messen des Antikörper immobilisierenden Abschnittes.

## Revendications

1. Procédé de dosage immunologique pour doser un antigène protéique, qui est l'hémoglobine A1c, le procédé comprenant les étapes de :
(a) fourniture d'un premier anticorps et d'un antigène protéique, qui est l'hémoglobine A1c, possédant, incorporé à l'intérieur, un épitope auquel le premier anticorps peut spécifiquement se lier ;
(b) mise en contact, avec l'antigène protéique, d'une IgM servant de second anticorps qui se lie à au moins une partie de l'antigène protéique à l'exclusion de l'épitope de manière à exposer l'épitope de l'antigène protéique ; et
(c) mise en contact du premier anticorps avec l'épitope de l'antigène protéique après l'étape (b).

2. Procédé de dosage immunologique selon la revendication 1, dans lequel le second anticorps est un anticorps monoclonal.

3. Procédé de dosage immunologique selon la revendication 1, dans lequel le premier anticorps est immobilisé sur un support solide.

4. Procédé de dosage immunologique selon la revendication 3, dans lequel le support solide est une plaque de microtitration.

5. Procédé de dosage immunologique selon la revendication 3, dans lequel le support solide est une bande de test.

6. Bande de test à utiliser dans un procédé de dosage immunologique pour doser un antigène protéique, qui est l'hémoglobine A1c, la bande de test comprenant :
un matériau de base ;
une section d'immobilisation d'anticorps pourvue sur le matériau de base avec un premier anticorps immobilisé dessus, le premier anticorps étant susceptible de se lier spécifiquement à un épitope situé à l'intérieur d'un antigène protéique, qui est l'hémoglobine A1c ;
une zone d'égouttement d'échantillon écartée de la section d'immobilisation d'anticorps sur le matériau de base ; et
une section de transmission d'impact située entre la section d'immobilisation d'anticorps et la zone d'égouttement d'échantillon sur l'élément de base, et écartée de la section d'immobilisation d'anticorps, la section de transmission d'impact étant imprégnée d'une IgM servant de second anticorps qui se lie à au moins une partie de l'antigène protéique à l'exclusion de l'épitope, de manière à exposer l'épitope de l'antigène protéique.

7. Appareil de dosage immunologique dans lequel une bande de test est introduite, la bande de test comportant :
un matériau de base ;
une zone d'égouttement d'échantillon ;
une section d'immobilisation d'anticorps écartée de la zone d'égouttement d'échantillon sur le matériau de base avec un premier anticorps immobilisé dessus, le premier anticorps étant susceptible de se lier spécifiquement à un épitope situé à l'intérieur d'un antigène protéique, qui est l'hémoglobine A1c ; et
une section de transmission d'impact située entre la section d'immobilisation d'anticorps et la zone d'égouttement d'échantillon sur l'élément de base, et écartée de la section d'immobilisation d'anticorps, la section de transmission d'impact étant imprégnée d'une IgM servant de second anticorps qui se lie à au moins une partie de l'antigène protéique à l'exclusion de l'épitope, de manière à exposer l'épitope de l'antigène protéique,
l'appareil de dosage immunologique comprenant :
une section d'égouttement d'échantillon pour faire tomber goutte à goutte un échantillon sur la zone d'égouttement d'échantillon ; et
une section de mesure optique pour mesurer optiquement la section d'immobilisation d'anticorps.
